# EUROPEAN PATENT SPECIFICATION

(11) **EP 2 572 700 B1**
(45) Date of publication and mention of the grant of the patent: **20.12.2017**
(21) Application number: 11783754.2
(22) Date of filing: 17.05.2011
(51) Int. Cl.: A61Q 7/00, A61P 17/14, A61K 8/55, A61K 31/685

(54) **COMPOSITION FOR PREVENTING HAIR-LOSS OR STIMULATING HAIR GROWTH**
ZUSAMMENSETZUNG ZUR VERHINDERUNG VON HAARVERLUST ODER ZUR STIMULATION VON HAARWACHSTUM
COMPOSITION POUR LA PRÉVENTION DE LA PERTE DE CHEVEUX OU LA STIMULATION DE LA POUSSE DES CHEVEUX

(30) Priority: 19.05.2010 KR 20100046940
(43) Date of publication of application: 27.03.2013
(73) Proprietor: Biopid Corporation, Chuncheon-si, Gangwon-do 200-822 (KR)
(72) Inventor: CHOI, Seong Hyun, Chuncheon-si Gangwon-do 200-822 (KR)
(74) Representative: RGTH
(86) International application number: PCT/KR2011/003652
(87) International publication number: WO 2011/145872

(56) References cited:
- EP-A2- 0 060 933
- WO-A1-92/12703
- WO-A1-2004/019900
- WO-A1-2006/091033
- WO-A1-2007/097502
- DE-A1- 4 113 346
- GB-A- 2 004 741
- JP-A- H0 193 510
- JP-A- S60 208 905
- JP-A- 2006 028 026
- KR-A- 20040 008 916
- NL-C1- 1 020 431
- US-A- 5 116 605
- US-A- 5 217 711
- US-A1- 2004 266 730
- US-A1- 2007 010 429
- US-A1- 2009 042 840
- ARMANDO JOSE YANEZ SOLER: "CULTURAL EVOLUTION AS A POSSIBLE TRIGGERING OR CAUSATIVE FACTOR OF COMMON BALDNESS", MEDICAL HYPOTHESES, EDEN PRESS, PENRITH, US, vol. 62, no. 6, 1 January 2004 (2004-01-01), pages 980-985, XP008048709, ISSN: 0306-9877, DOI: 10.1016/J.MEHY.2003.12.031
- TOMOYA TAKAHASHI ET AL.: 'Phosphatidic acid has a potential to promote hair growth in vitro and in vivo, and activates mitogen-activated protein kinase/extracellular signal- regulated kinase kinase in hair epithelial cells' J. INVEST. DERMATOL. vol. 121, no. 3, 2003, pages 448 - 486, XP002482783

## Description

### [Technical Field]

The present invention relates to a composition for preventing hair loss or stimulating hair growth, including phospholipids as an effective component, and more particularly, to a composition for preventing hair loss or stimulating hair growth, wherein the phospholipids are extracted from an animal lung, as an effective component.

### [Background Art]

Excessive male hormone action, poor blood circulation, a scalp function decrease due to peroxides, bacteria, and the like, a genetic factor, aging, stress, and the like are being considered as the cause for hair loss, but the obvious cause of hair loss is not known up to now. Recently, the percentage of the population that is worrying about hair loss is increasing, the age is continuing to decline, and the percentage of the woman population that is worrying about hair loss is gradually increased, because of nutritional unbalance due to a change of dietary life, increased levels of stress due to environmental pollution and the social environment, and the like.

For preventing or treating such hair loss, formulations having a female hormone as a main component for stimulating blood circulation, reinforcing hair follicle functions, moisturizing scalp, and suppressing a male hormone, or formulations having minoxidil, finasteride, and trichosaccharide, and the like, had already developed. A functional mechanism of minoxidil on effect of hair growth is still unclear, but it is considered to induce hair growth by an increase of nutrient supply through a vasodilatation, and an effect of potassium channel opening. The finasteride is a 5-α reductase inhibitor, and being used as a medicine for treating a prostate and a medicine for preventing hair loss through varying dosage. The finasteride is a good drug with respect to convenience and effectiveness. However, since regents for preparing the finasteride are expensive, or highly toxic, there are problems that cost of manufacture is high, and the environment is contaminated. Especially, there are problems that since by-products are difficult to be removed, a purity of object material may be decreased, and it is difficult to produce in bulk because regents or active derivatives, in which activity can be easily inhibited by water, is used.

EP 0 060 933 A2 discloses a composition for stopping of hair loss and promoting new hair growth. The active ingredients are vegetable lecithins, as well as cytochromes, phosphatidyl inositols, phosphtides and phosphatidic acids. Apart from the vegetable lecithins the other ingredients are obtained by extraction from fresh animal hearts.

WO 2007/097502 A1 discloses a composition for reducing the exudation of serum proteins. The composition is used for the treatment of certain conditions by reducing the exudation of serum proteins in skins and mucosae, wherein the conditions include atopic dermatitis, atopic eczema, contact dermatitis, asthma etc.

### [Disclosure]

### [Technical Problem]

The present invention is designed in order to solve the above-mentioned problems, and by the above-mentioned needs, and an object of the present invention is to provide a composition for preventing hair loss or stimulating hair growth.

### [Technical Solution]

In order to achieve the above object, an exemplary embodiment of the present invention provides a composition for preventing hair loss or stimulating hair growth, in which the composition includes phospholipids extracted from an animal lung as an effective component. The phospholipids are extracted from animals, and the animals are preferably livestock, for example, cows or pigs, but the present invention is not limited thereto.

According to an embodiment of the present invention, the phospholipids are preferably extracted from a washing solution of lung, or a broken material of lung tissue, but the present invention is not limited thereto. The phospholipids are "phospholipids having a degree of saturation, and relatively short hydrocarbon (tail) length of fatty acid", and more preferably, the hydrocarbon length is 14 to 16, but the present invention is not limited thereto.

For example, the phospholipids include any one selected from the group consisting of dipalmitoylphosphatidylcholine, phosphatidylcholine, phosphatidylserine, phosphatidic acid, and phosphatidylinositol.

According to a preferable example of the present invention, more preferably, the composition includes divalent cation and carboxyl group-containing organic acid, or salt thereof, but the present invention is not limited thereto.

Hereinafter, the present invention will be described.

The present invention suggests a new method for solving hair loss through considering literatures about a common biochemical and physiological phenomenon that is shown in all the symptoms of hair loss, such as a male-type hair loss and female-type hair loss. A new consideration and solution way for hair loss are as follows:
Yanez Soler suggested hypothesis disclosed in his thesis that hair loss is caused by flowing and entering sebum secreted from sebaceous grand into follicular or dermal papilla, even though the sebum should be secreted to the outside by getting on hairs. He supposed that in the case of having a condition to easily discharge the sebum along hair shaft to the outside, for example, as a thickness of hair is thick or a density of hair is high, hairs are easily not lose. One excellent example explaining his hypothesis is phenomenon that the area to be contacted to a pillow dose not exhibit hair loss. It is because while humans take sleep, hairs are squashed due to a pillow and connected to each other, so that sebum is easily removed along surface of hairs (Yanez Soler 2004. Med. Hypotheses 62: 980-985).
* The present invention supposes that a fundamental cause of hair loss is deeply involved in an excessive secretion and accumulation of sebum constituted of neutral fat components. Based on the above supposition, we attempt to explain all the causes of hair loss. Especially, we attempt to simply explain the reasons that many males suffer with hair loss with age. The physiological and biochemical causes of hair loss have commonality and theory rationality from the documents as follows:
   - Androgen, a male hormone, is deeply involved in an increase of triacylglycerol (TG) and cholesterol, which are a representative neutral fat in the body (Kao et al. 2001. J. Int. dermatol. 116: 443-451).
   - A rate of neutral fat is increased as compared with polar lipid in the body with age (Nazzaro-Porro et al. 1979. J. Invest. Dermatol. 73: 112-117).
   - A secretion of sebum and activity of sebaceous grand are deeply involved in a level of male hormone according to age (Pochi et al. 1979. J. Invest. Dermotol. 73: 108-111).
   - A synthesis of cholesterol, a representative neutral fat, is increased due to stress (O Donnell et al. 1987 J. Royal Society Med. 80: 339-341).
   - After some cancer patients are treated with a chemical therapy, a neutral fat (especially, triacylglycerol), a low density lipoprotein (LDL), and LDL-cholesterol are increased (Alexopoulos et al. 1992. Cancer. Chemother. Pharmacol. 30: 412-416).
   - In a case in which an unsaturated fatty acid of lipid constituting sebum or epidermis surroundings is under oxidative stress by singlet oxygen, ¹O₂, produced through a lipid peroxidation process, hair loss may be generated (Naito et al. 2008. International J. Mol. Med. 22: 725-729).
   - Some patients suffered from hair loss may exhibit microinflammation. An excessive sebum secretion is possible to give good enviornment for attaching and proliferating microorganisms (Mah@et al. 2000. International J. Dermatol. 39: 576-584).

From the above-listed scientific basis, it could easily be deduced that a ratio of a neutral fat in the body may be increased due to a male hormone, stress, and aging, and an excessive sebum secretion and synthesis generated by the neutral fat component may cause a symptom of hair loss. In the case of causing hair loss due to genetic factors, it could be deduced that when having the genetic factors toward a mechanism of a neutral fat synthesis, an imbalance between ratio of a polar fat and a neutral fat is induced, and thus hair loss may be stimulated. Especially, it can be anticipated that since mitochondria mainly plays a role involving to a lipid mechanism, a baldness inheritance is a maternal inheritance that is a transfer route of mitochondria. It is considered that baldness may be generated by any mutation or imbalance of lipid mechanism relating to mitochondria.

It is considered that as the reason that females do not exhibit male-type hair loss, females exhibit a relatively low secretion of sebum physiologically, also generally, they completely wash their faces as compared with male, relatively, and their sebum of neutral fat component is effectively removed by using various cosmetics at the time of washing their faces. In addition, it could be deduced that various fatty acids, fatty acid derivatives, and various emulsifier components included in cosmetics prevent neutral fat sebum from having high viscosity, so that they do not exhibit male-type hair loss symptom.

In addition, difference between Asian having small hairs and Westerner having large hairs can be generally explained with the same reasons. For Asian ingesting mainly carbohydrates, when accumulation of fat in the body begins to start, neutral fats that is a type of store is easily increased, so that a hair development of Asian is less effective as compared with Westerner. Such phenomenon can be explained with the same reason.

When sebum, a neutral fat, is not discharged to the outside of the body along hairs, but transferred to hair follicles, and then accumulated, there are problems at hair follicle cells and surroundings cells that are supplied with nutrients from capillary. Since nutrients required for cell proliferation and activity are supplied in a type of micelle or liposome (HDL or LDL, secretary vesicles or lamellar bodies, and the like), if sebum, a neutral fat, is accumulated around the hair follicle cells, a delivery of the nutrients that are supplied in a state of being surrounded with lipid layer, and the like, are interrupted. In addition, since a neutral fat pushes water (moisture), a delivery of small watersoluble molecules (glucose, amino acid, and the like) to hair follicle cells is blocked. It can be described that miniaturization phenomenon of hair follicle that is exhibited commonly when hair loss happened occurs by contracting cell activity and cell size, and then eliminating cells through inhibiting hair follicles and all relevant surrounding cells from being supplied with good nutrient from blood vessel. In addition, it can be deduced that if hairs are lost one time according to a cycle of hair follicle cells, new hairs should be again growth, but accumulation of a neutral fat inhibits a delivery of stem cells producing hair follicles to dermal papilla, thereby inhibiting a formation of new hair.

Recently, 20s to 30s young males suffered from hair loss are increasing. A cause for Hair loss of young males can be explained in connection with metabolism of neutral fats as follows. In the early 1980s, vegetable oils (soybean oils, corn oils, grape seed oils, cottonseed oils, olive oils, and the like) were distributed in bulk at a low price, so that in various dietary life, intake of fried food or oil food were increased. Such commercialized vegetable oils are mainly produced in bulk by using an extraction method with a neutral solvent (for example, n-hexane), and the like. Since the extraction method using a neutral solvent does not give a condition or chance to be included in food to polar lipids included in plants, clear and transparent oil composed of almost pure neutral fats comes into the market. As a result, it can be deduced that modern people intake many vegetable neutral fats including unsaturated fatty acids, have a high level of dependence on metabolism of neutral fats, and also have a high level of accumulation of neutral fats in the body and secretion of sebum of neutral fat components. As disclosed above, excessive neutral fats may cause hair loss. In addition, since neutral fats derived from plants include many unsaturated fatty acids, when exposed in the air, the neutral fats have to be gone through a serious chemical reaction, referred to as "lipid peroxidation" by combining with oxygen molecular when exposed in the air. Oxidation stress generated by peroxidation process may be a cause for accelerating hair loss. Hair loss of young people may occur by an individual mental stress, but hair loss is possible to be explained by a serious of physiological functions relating to neutral fats. In addition, it is assumed that there is a possibility that part of vegetable unsaturated fatty acids is changed to arachidonic acid thereby amplifying inflammatory reaction.

When a secretion of sebum having excessive neutral fat components and a reverse movement of sebum to hair follicles cause hair loss, the symptom of hair loss can be alleviated by using traditional methods that are conventionally known. For example, they can be adopted, such as a method for decreasing sebum synthesis and sebum secretion using a method for inhibiting androgen activity, or a method for decreasing androgen activity using an inhibitor for preventing androgen synthesis from cholesterol. For example, there are methods for using minoxidil, peripheral vasodilators and finasteride, 5α-reductase, as a hair growth solution. However, the above-described both drugs are known to obtain an effect when using them for a long period of time, and also it is known that they are ineffective for preventing hair loss in the cases of some users. It is reported that long-term use of the finasteride may cause side effects such as sexual dysfunction, hypospermia, and a weak sexual appetite (Kaufman et al. 1998. J. Am. Acad. Dermatol. 39: 578-589), and the minoxidil may be accompanied by symptoms such as contact dermatitis, and eczema, or may induce headache for some patients (Oslen et al. 2002. J. Am Acd. Dermatol. 47: 377-385).

As the other methods for preventing hair loss, there is a method for continuously removing sebum using detergents, shampoos, or the like having a strong washing effect. A continuous sebum-removing method has potential to prevent sedum from being reverse moved to hair follicles. However, there are chances that if using a strong detergent or shampoo, a chemical stimulation may be generated to scalp or epidermis, or a protective function that is their natural function may be deteriorated thereby generating opposite effects.

Therefore, it may be a stable method that instead of methods for decreasing a production or secretion of sebum, or removing accumulated sebum, simply, sebum properties are converted to be easily discharged thereby preventing hair loss and also the conditions for easily supplying nutrients to hair follicles cells and surrounding cells are generated to stimulate hair growth.

According to the present invention, it was found that when topically applying the composition including the phospholipids that are purified after extrating from animal lungs, divalent cation, and organic acid (as for preventing phospholipids crumpling phenomenon generated by pH buffer solution using organic acid and organic acid salt and divalent cation; for example, citric acid and citric acid salt, pH 5.8) to epidermis of mouse, the composition can stimulate differentiation of hair follicle and hair growth in addition to alleviation of epidermis inflammation.

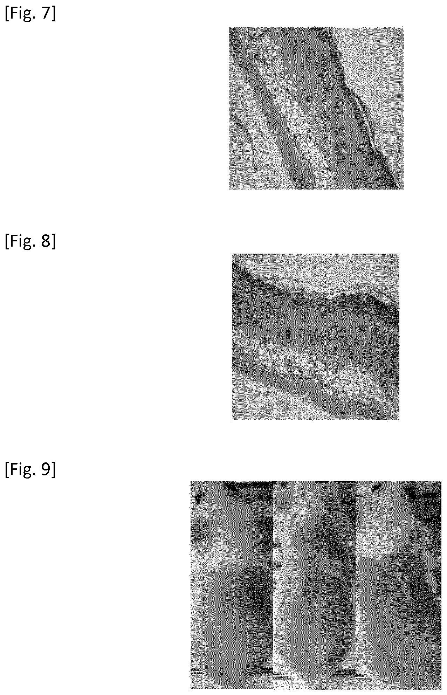

A result observed from the present invention shows activation of hair follicle cells in a different type observed from the conventional results (typical process of hair follicle formation, that is, budding of basal cell layer of epidermis initiates, and then follicle is formed in the new bulge). From a result of observing histological examination, it could be confirmed that an existed hair follicle cell distributed at the hypodermis layer is quickly differentiated and grown, and a great number of hair follicles are produced as compared with the number of results disclosed in the conventional thesis (see Figs. 7 and 8). From the above results, we considered the function of the composition for hair growth, and then found new theory about the causes of hair loss.

Accordingly, the present invention is a composition for preventing hair loss and stimulating hair growth. The present invention founds new theory about the causes of hair loss and suggests a composition for preventing and stimulating hair growth as a new concept. A composition containing phospholipids purified through removing neutral fats and proteins from lung tissue of animals, divalent cation (calcium ion and/or magnesium ion), and/or organic acid (for example, citric acid and citric acid salt) stimulates hair growth. The present invention is characterized by using specific phospholipids that are secreted from lung tissues of animal and human to the outside of the body, not like the phospholipids (generally, referred to as 'lecithin") extracted from egg or soybean, in which since the phospholipids secreted from animal or human has stability about oxidation of lipid, when applied on the site of scalp and epidermis, that are exposed with air, it is favorable.

The term, "extract" used in the present specification has the meaning that is commonly known as a crude extract in the art as described above, but includes the above described solvent fractions in a broad sense.

The lung extract of the present invention is obtained by using solvent, and also obtained by further applying a purification process. For example, the fractions obtained through a ultrafiltration membrane having a certain molecular cut-off value from the extract, and fractions obtained through various purification methods further performed, such as an isolation by using various chromatographs (manufactured for isolating according to size, charge, hydrophobic or affinity) are included as the lung extract of the present invention.

The lung extracts according to the present invention, or fractions thereof may be prepared in a type of powder through a further process, such as vacuum distillation, and freeze-drying or spraying drying.

The composition of the present invention functions very effectively on preventing hair loss or stimulating hair growth. The term "hair loss prevention" and "hair growth stimulation" used in the present specification are used as the same meaning, and have the same meaning as the other term, hair growth used in the art.

According to a preferable embodiment of the present invention, the composition of the present invention may be provided as a pharmaceutical composition, a cosmetic composition, or a food composition.

In a case where the composition of the present invention is prepared as a pharmaceutical composition, the pharmaceutical composition of the present invention includes a pharmaceutically acceptable carrier. The pharmaceutically acceptable carrier included in the pharmaceutical composition of the present invention includes, as one generally used at the time of preparing, lactose, dextrose, sucrose, sorbitol, mannitol, starch, acacia rubber, calcium phosphate, alginate, gelatin, calcium silicate, microcrystalline cellulose, polyvinyl pyrroidone, cellulose, water, syrup, methyl cellulose, methyl hydroxyl benzoate, propylhydroxy benzoate, propylhydroxy benzoate, talc, stearic acid, magnesium and mineral oil, but the present invention is not limited thereto. The pharmaceutical composition of the present invention may further include a lubricant, a wetting agent, a sweeting agent, emulsion, suspension, preservatives, and the like. The suitable pharmaceutically acceptable carrier or formulations are described in detail in Remington's Pharmaceutical Sciences (19th ed., 1995).

The pharmaceutical composition of the present invention may be orally or parenterally administrated, preferably parenterally administrated, and more preferably applied in a type of topical application by applying.

A proper dosage of the pharmaceutical composition according to the present invention may be variously prescribed according to factors such as a formulation method, an administration method, age of a patient, body weight of a patient, sex of a patient, a pathosis of a patient, food, an administration period, an administration route, an excretion rate, and reaction sensitivity. A dosage of phospholipids, a bioactive substance, included in the pharmaceutical composition of the present invention is within a range of 0.001 to 1000 mg/kg, preferably 0.001 to 100 mg/kg, and more preferably 0.01 to 50 mg/kg based on an adult.

The pharmaceutical composition of the present invention may be prepared in a type of unit capacity or by putting in high capacity container through preparing with a pharmaceutically acceptable carrier and/or excipient according to the method that can be easily performed by a person skilled in the art relating to the present invention. At this time, the dosage form may be a type of solution, suspension, syrups, or emulsion in an oil or aqueous medium, or may be a type of extracts, discutient, powders, granulars, tablets, or capsules. In addition, it may further include a dispersant or a stabilizer.

In a case where the composition of the present invention is prepared as a cosmetic composition, the composition of the present invention includes the above-described lung extract and phospholipids isolated from the same, and also the components that are generally used in the cosmetic composition, in which the components include for example, general adjurvants, such as an antioxidant, a stabilizer, a dissolving agent, vitamins, pigments, and flavouring, and carriers. In addition, the composition of the present invention may use a mixture mixed with a hair growth promoter and a hair tonic that are conventionally used within the range in which the function (hair growth function) of the present invention is not damaged, in addition to the above-described lung extract and phospholipids isolated from the same.

As the carriers, purified water, monovalent alcohols (ethanol or propyl alcohol), polyvalent alcohols (glycerol, 1,3-butylene glycol, or propylene glycol), high fatty acids (palmitic acid or linolenic acid), fats (wheat germ oil, camellia oil, jojoba oil, olive oil, squalene, sunflowers oil, macadamia nuts oil, avocado oil, soybean water-added lecithin, or fatty acid glyceride), and the like may be used, but the present invention is not limited thereto. In addition, if necessary, a surfactant, an antimicrobial agent, an antioxidant, an ultraviolet ray adsorbent, anti-inflammatory, and a refrigerant may be added.

The surfactant may include one selected from the group consisting of polyoxy ethylene, hydrogenated castor oil, polyoxy ethylene, oleyl ether, monooleic acid polyoxyethylene, polyoxy ethylene, glyceryl monostearate, monostearic acid sorbitan, monooleic acid polyoxy ethylene, sorbitan, sucrose fatty acid ester, monolauric acid hexaglycerin, polyoxy ethylene reduced lanolin, POE, glyceryl pyroglutamic acid, isostearic acid, diester, N-acetylglutamin, and isostearyl ester.

The antimicrobial agent may include one selected from the group consisting of hinokithiol, triclosan, chlorhexidine gluconic acid salt, phenoxy ethanol, resorcin, isopropylmethylphenol, azulene, salicylic acid, and zincpyritaon.

As the antioxidant, any one from butylhydroxyanisol, gallic acid, propyl gallate, and erythorbate may be available.

As the ultraviolet ray absorbent, any one from benzophenones such as dihydroxybenzophenone, melanin, paraaminobenzoic acid ethyl, paradimethyl aminobenzoic acid 2-ethylhexyl ester, cynocite, paramethoxy cinnamic acid 2-ethylhexylester, 2-(2-hydroxy-5-methylphenyl) benzotriazole, urocanic acid, and metallic oxide particles may be available.

As the anti-inflammatory, glythylic acid dipotassium or allantoin may be used, and as the refrigerant, capsicum tincture or 1-menthol may be used.

The dosage form of the composition is an arbitrary dosage form that is capable of combining the lung extract and phospholipids isolated from the same as an effective component, and as a type of cosmetic for preventing hair loss or stimulating hair growth, it can be prepared in various types, such as hair tonic, hair aerosol, pomade, powder, a solution such as a gel, solgel, emulsion, oils, wax, and aerosol, but the present invention is not limited thereto.

The composition of the present invention may further include an assistance component for stimulating hair growth. The assistance component for stimulating hair growth includes 5α-reductase inhibitor, hair follicle and hair follicle activator, hair follicle blood flow increasing component, a sterilization component, a dandruff prevention agent, a keratin softener, a refrigerant, a moistener, and the like. Examples of components about each function are as follows:
1) 5α-reductase inhibitor
   Green tea extracts, finasteride, thujae orientalis seed extracts, sophora angustifolia root extracts, linoleic acid, gamma linoleic acid, jojoba oils, olive oils, sunflower oils, abocado oils, soybean added lecithin, clove extracts, coix seed extracts, rhus semialata gall extracts, turmeric extract, betel nut extract, uncaria gambir extracts, morning glory seed extracts, plantain seed extract, dalbergia odorifera extracts, licorice extracts, bogolji extracts, saw palmetto, apple extracts, and the like.
2) Hair follicle and hair follicle activator
   Swertia extracts, ginseng extracts, red ginseng extracts, actinidia arguta fruit extracts, clove extracts, coix seed extracts, rehmannia glutinosa root extracts, polygonum multiflorum root extracts, polygonatum sibiricum extracts, pantothenylethylether, dipantothenol, henna extract, acorus calamus extracts, hinoki thiol, nicotinamide, L-menthol, and the like.
3) Hair follicle blood flow increasing component

   Minoxidil, ginkgo extract, red ginseng extracts, capsicum tincture, tocopherol acetate, nicotinic acid benzyl, and the like.
4) Sterilization and keratin dissolving agent
   Salicylic acid, henna extracts, calamus extract, piroctone olamine, and the like.
5) Dandruff prevention agent
   Ketoconazole, green tea extracts, and the like.
6) Refrigerant
   L-menthol, and the like.
7) Moistener
   Olive oil, jojoba oils, camellia oil, sunflower oil, macadamia oil, avocado oil, and the like.

### Hereinafter, the present invention will be described in detail.

Solutions about hair loss, which is suggested in the present invention, are as follows:

### 1. A property of sebum, a neutral fat, is changed by using a polar fat.

Phospholipids, a representative polar fat, are a specific amphiphilic material, and can be well mixed with a neutral fat, and at the same time can be well mixed with water. A property of sebum is changed by using such a property of the phospholipids. Sebum is well mixed with water, so that sebum is easily discharged and it is possible to not flow sebum backward to hair follicles and not accumulate sebum. Therefore, the accumulated sebum dose not blocks nutrients to be transferred to blood vessels. The phospholipids are lipids constituting cell membrane, and can be supplied as a membrane building block required for hair follicle cells and surrounding relevant cells. A soap or shampoo can relatively easily remove fatty acids or cholesterols that have a bit polarity among sebum components, but it is difficult to remove complete neutral fats, such as triacylglycerol or cholesterol ester. Accordingly, if a soap or shampoo is frequently used, rates of fatty acids, cholesterol, and the like included in sebum are gradually decreased, so that there are worries that solidification can be generated. Since the method of changing properties of sebum using phospholipids prevents the solidification, it is expected that sebum is easily discharged, and also easily removed at the time of using a soap or shampoo.

### 2. The phospholipids should be selected and then used.

Since vegetable phospholipids include a great quantity of unsaturated fatty acids, when contacted with oxygen by being exposed in air at the time of applying on scalp or epidermis, they are subjected to a lipid peroxidation process. A singlelet oxygen (¹O₂) radical generated by the lipid peroxidation process may negatively affect (oxidative stress) epidermal cells and hair follicle cells. It was previously reported that the lipid peroxidation may cause hair loss (Naito et al. 2008. International J. Mol. Med. 22: 725-729) or alopecia areata symptom (Koca et al. 2005. Med. Sci. Monit 11: 296-299).

In order to use the phospholipids on epidermis of the body, the content of unsaturated fatty acids is low or the unsaturated fatty acids are not included in the phospholipids. Lung that contacts highly with oxygen physiologically is only an organ including mostly unsaturated fatty acids. It is considered that such a property of lung is a method for lowering a surface tension of lung surface in order to easily breathe, and also since a continuous exposure to oxygen is inevitable while breathing, such a property of lung is one of evolution results for being protected from an adverse effect of lipid peroxidation. As a result, the lungs of all the animals including human has the most "phospholipids including a great amount of saturated fatty acids," and continuously secret such phospholipids on the surface of lung. Accordingly, the phospholipids derived from the animal lung are used as the phospholipids that are capable of being applied on epidermis or scalp.

There are cases of using a plant-extracted oil or specific fatty acid as some products relating to hair loss. The plant-extracted oils or plant-derived fatty acids can allow sebum liquefaction (increase of fluidity). In addition, they can exhibit an effect on preventing hair loss or stimulating hair growth by easily discharging sebum. However, it is not well considered about an opposite effect caused by unsaturated fatty acid-oxidation.

### 3. Co-factor, divalent cation for maximizing a phospholipid effect.

It is difficult to prevent hair loss or expect fast hair growth by simply supplying phospholipids to scalp. Divalent cations, especially, calcium ion (Ca²⁺ ion), is essential for supplying the phospholipids supplied from the outside to dermal papilla and relevant surrounding cells. In general, calcium ion (Ca²⁺ ion) or magnesium ion (Mg²⁺ ion) is used for a cell-cell recognition, and is an essential factor for a fusion (or integration) of micelle or liposome to a selected cell membrane. Divalent cations should be essentially co-existed in order to exhibiting an effect of the selected phospholipids in a living body. In addition, it is expected that such divalent cations neutralize a negative polar of phosphate group of the phospholipids, so that the phospholipids can determine their orientation, or have a stable structure. A concentration of calcium, divalent cation used for non-limitation examples of the present invention imitates a calcium ion concentration of lung surface in a living body, and then is applied.

### 4. Co-factor, organic acid for an effective function of phospholipids

The phospholipids exhibit a strong aggregation phenomenon by divalent cation because of lipid polar head having a strong polar. The phospholipid aggregated by divalent cation is not easily dispersed on epidermis, and since the size of the aggregates is large, it is not easily penetrated into epidermis, so that it is difficult to expect a great effect on preventing hair loss and stimulating hair growth. In order to minimize a random aggregation of phospholipids by divalent cation, an organic acid (for example, organic acids relating to TCA cycle, such as citrate/citric acid or succinate/succinic acid) exhibiting a strong negative polar can be used in the composition. Such organic acids play a role in involving an interaction of phospholipids aggregation by divalent cation. Such a role is a different way of fixing divalent metal ion by ethylenediaminetetraacetic acid (EDTA), a representative chelating chemical. In addition, there is an advantage that an organic acid presents in a type of conjugated acid or conjugated base (for example, citric acid and sodium citrate), so that the organic acid can be used as a buffer solution for controlling pH of liquid preparation applied on scalp.

### Originality of the present invention

Examples of methods for stimulating hair growth using phospholipids are disclosed in the conventional patients.

US patent No. 4,828,837 and US patent No. 4,761,288 disclose liposome prepared by using phospholipids in order to maximize an effect of minoxidil. Such patents use the liposome encapsulating minoxidil using phospholipids (lecithin) and dipalmitoyl phosphatidylcholine (DPPC) synthesized, soy, or egg York. In addition, for stabilizing liposome structure, such patents suggest experimental examples further using cholesterol. An object of the present invention is to stimulate hair growth using the purified phospholipids after extracting animal lungs and then purifying, but not to deliver a component stimulating hair growth, such as minoxidil. With a universal knowledge, it is difficult to make a decision that the phospholipids, a constituent component in a living body, stimulate stem cells that are distributed on stratum basal, so that the stem cells can be differentiated into hair follicle cells. Therefore, a principle of the present invention is to prevent sebum solidification and easily discharge or remove sebum by changing a property of sebum, a neutral fatty component, using the pure phospholipids purified from the animal lung tissues. A smoothly sebum elimination prevents hair loss, and helps that hair follicle cells can easily receive nutrients from capillary vessel, so that it can activate hair follicle cells and relevant surrounding cells thereby stimulating hair growth.

The phospholipids extracted from egg York or soy include many unsaturated fatty acids, so that there is high probability of giving oxidative stress to hair follicle cells and relevant surrounding cells by easily peroxidating through an oxygen contact. Therefore, if possible, it is preferable that the phospholipids purified from animal lung including many saturated fatty acids be used instead of using the phospholipids (lecithin) including unsaturated fatty acids.

The present invention relates to a composition using with organic acid or organic acid salt (conjugated acid or conjugated base) in order to prevent an aggregation of phospholipids caused by calcium, along with divalent cation. Therefore, the present invention has originality that is completely different from any conventional phospholipids-related products. By using such divalent ion, and organic acid and organic acid salt at the same time, it is unfavorable to prepare structurally stable liposome. The reason is that head groups of phospholipids having strong polar push polarity to each other. As the content of dipalmitoyl phosphatidylcholine (DPPC) is high, the structurally stable liposome is unfavorable. As an example, it is reported that when a pure DPPC liposome is stored at 4 degrees, it is completely broken within 2 weeks (Launois-Surpas et al. 1992. Colloid Polym. Sci. 270:901-911).

The present invention prefers to use phospholipids in a type of a simple suspension in order to change a property of sebum presenting on scalp or epidermis. It is because when a deliver structure of liposome type is table, it is apprehended that it is not easily mixed with sebum exposed in scalp or within the sebaceous glands.

A method of using phospholipids in order to change a property of sebum has more preferable advantage than hair growth solution using various fatty acids (US patent No. US2007/0154432 A1). It is because the fatty acid may be a bad influence on cell lipid membrane structure, and also may be easily used as an energy source of bacteria or fungi. In addition, an intermediary metabolite of fatty acid has a property exhibiting a bad smell (for example, butyric acid, propionic acid, or the like), so that there are many disadvantages. Furthermore, linoleic acid is changed into arachidonic acid by a metabolism through human cells, and then finally into leukotriene-B4 (LTB4) or prostaglandin E2. Such two materials play a role in increasing inflammatory reaction, so that when using them for a long period of time, this has potential to stimulate hair loss (Mahet al. 2000. International J. Dermatol. 39: 576-584).

Hair growth products using fatty acid may exhibit initially a hair growth effect by softening a property of sebum through a well fusion with sebum. However, it cannot be ignored that when accumulated because of using them for a long period time, this has potential to exhibit a negative function.

As a component in the composition of the present invention, the composition includes pure phospholipids that are obtained by removing neutral fats and peptides (proteins) from animal lungs, divalent cation (for example, calcium ion and/or magnesium ion), organic acids (for example, citric acid/citrate, succinic acid/succinate, or malic acid/malate), and organic acids (conjugated acid and conjugated base) that are used in tricarboxylic acid cycle of mitochondrial. In addition, an object of the present invention is to easily remove or discharge sebum through a change of property of sebum that is secreted from sebaceous glands, and then to prevent hair loss and stimulate hair growth.

### Various application of the composition

As for the composition of the present invention, there is no basis or insufficient basis that the composition gives any effect or stimulation to stem cells distributed on epidermis to form new hair follicle cells and then stimulate hair growth, theoretically or experimentally. However, it was verified theoretically or experimentally that the present composition changes a property of sebum to easily discharge or remove sebum, and consequentially, stimulates hair growth, quickly.

As a result of finding a mechanism of hair growth through the conventional animal experiments, it was found that a differentiation and generation of hair follicle cells are naturally generated after being wounded and then treated (Cheng-Ming Chuong, 2007. Nature 447: 265-266 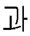 Ito et al. 2007. Nature. 447 316-320). When the composition of the present patient is applied on minor injury after making the minor injury, the hair follicle cells differentiated newly during a process of wound healing can be quickly grown and then can stably grow hairs. An object of stimulating hair growth is stronger than an object of preventing hair loss.

As another method, it is expected that in the case of exhibiting finely hair growths after applying minoxidil or finasteride, when the composition of the present invention is applied, hair growth can be quickly induced thereby planning a stabilization of hair growth.

In a case where fine hairs (called as to fine soft hairs) present on scalp among the people under the progress of hair loss, it is expected that the composition of the present invention is applied on the scalp, so that it can help an alleviation of hair loss symptom through quickly progressing the fine hairs.

Furthermore, it is expected that before and after performing a chemical therapy for patients suffered from cancer, when the composition of the present invention is applied, hair loss due to a side effect of the chemical therapy can be prevented, and also new hair growth can be quickly progressed when new hair growth is progressed after completing the chemical therapy, so that the composition can help a recovery for the patient.

### [Profitable effect]

As can be seen from the specification of the present invention, the present invention can prevent a solidification of sebum and easily discharge or remove sebum by changing a property of sebum, a neutral fat component using phospholipids purified from animals. A smooth elimination of sebum can prevent hair loss, and help that hair follicle cells receive easily nutrients from capillary vessel to activate hair follicle cells and relevant surrounding cells, thereby quickly growing hairs.

### [Description of Drawings]

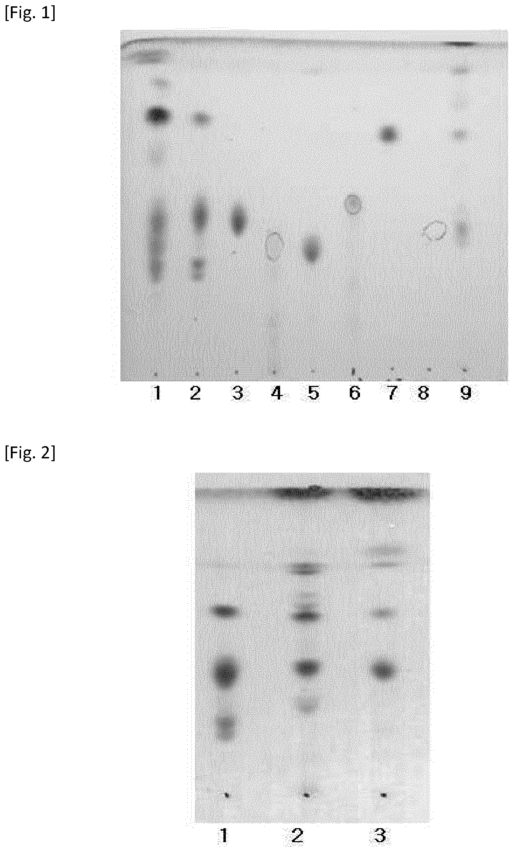

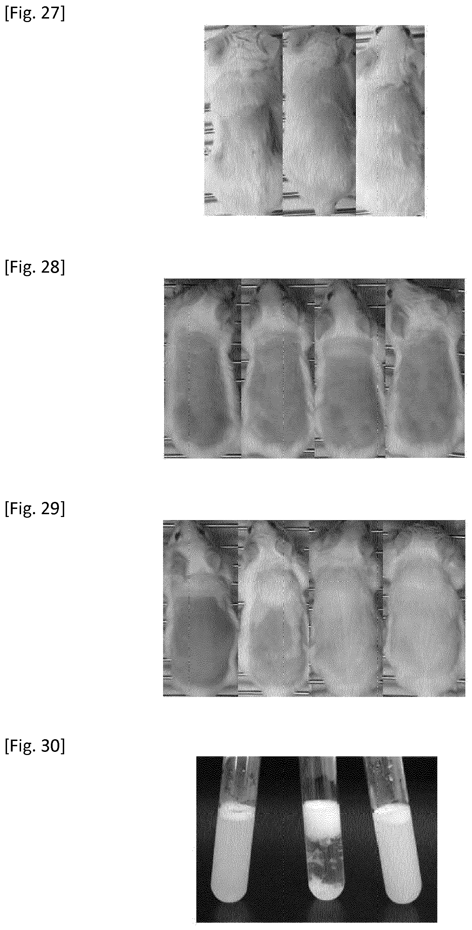

Fig. 1 is a photograph illustrating a thin layer chromatograph analysis of phospholipid extracted from pig lung and purified. In Fig. 1, a sample in lane 1 is a phospholipid extracted from pig lung before a silica column; a sample in lane 2 is a purified phospholipid extracted pig lung; a sample in lane 3 is a PC (L-alpha-phosphatidylcholine, Sigma #P3556); a sample in lane 4 is a PI (phosphatidylinositol sodium salt, Fluka #79403); a sample in lane 5 is a PS (3-SN-phosphatidyl-L-serine sodium salt, Sigma#P5660); a sample in lane 6 is a PA (3-SN-phosphatidic acid sodium salt, Sigma#P9511); a sample in lane 7 is a PE (L-alpha-phosphatidyl ethanolamine, Sigma#P7943); a sample in lane 8 is a DPPC (1-2-dipalmitoyl-sn-glycerol-3-phosphocholine, Sigma#P4329); and a sample in lane 9 is a soy lecithin (Junsei Chemical #86015-1201, Japan). A type of specific phospholipid purified was confirmed by using a thin layer chromatograph (TLC). A phospholipid confirmed by using the thin layer chromatograph was DPPC, PC, PS, PI, and PE. A developing solvent for the thin layer chromatograph is chloroform: methanol: water (65:25:4, volume ratio). After spraying 5% sulfate solution dissolved in ethanol, it was heated at 190 degrees to carbonize and confirm each component.
In Fig. 2, a sample in lane 1 is a phospholipid purified from pig lung; a sample in lane 2 is a soybean extraction phospholipid (soy lecithin, Junsei Chemical #86015-1201, Japan); and a sample in lane 3 is an egg York extraction phospholipid (egg lecithin, Tokyo Chemical Industry #L0022, Japan). From the method of thin layer chromatograph, it was confirmed that the phospholipid extracted from pig lung has a completely different constitution from the conventional well known phospholipid.

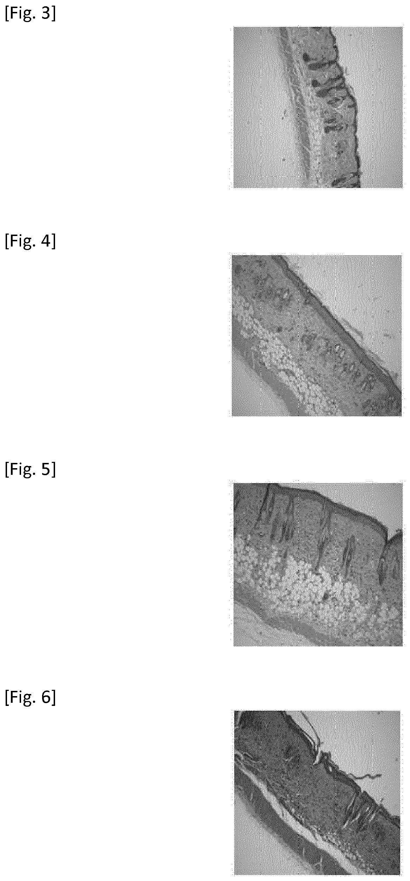

Figs. 3 to 8 are photographs confirming an activation phenomenon of hair follicle.
   An experiment for confirming an inflammatory decrease was performed by applying phospholipids extracted from pig lung on a back surface of mouse caused with an inflammation using DNFB (2,4-dinitrofluorobenzene) sensitization function. As a result, it was accidentally confirmed that the growth of hair follicle cells was activated.
   Fig. 3 is an epidermis tissue of a normal back; Fig. 4 is an epidermis tissue of mouse treated with only DNFB; Fig. 5 is an epidermis tissue after applying tacrolimus (Trade name, Protopic: Astella pharma Japan, Inc.); Fig. 6 is an epidermis tissue after applying dexamethasone (0.1% dexamethasone ethanol solution); Fig. 7 is an epidermis tissue after applying phospholipids (10 mg/ml) extracted from pig lung; and Fig. 8 is an epidermis tissue after applying phospholipids (20 mg/ml) extracted from pig lung phospholipids (10 mg/ml) extracted from pig lung, for 15 days as an application day. There were many activated hair follicle cells inside an oval red dotted line. A typical type of hair loss (phenomenon exhibiting cell basal layer dent and differentiation of hair follicles) (an application of tacrolimus in Fig. 5) was partially observed. After applying the composition of the present invention (Figs. 7 and 8), there were activated hair follicle cells under the cell basal layer, in which the activated hair follicle cells shows other type of hair growth as compared with the conventional type of hair growth.
Figs. 9 to 11 are photographs illustrating an effect of the composition of the present invention on growth stimulation of mouse hair.

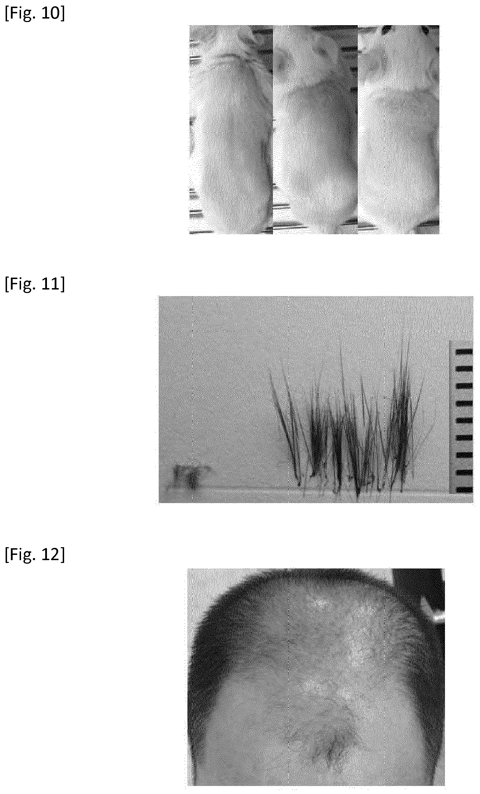

   Fig. 9 is photographs illustrating a result after applying saline solution for 2 weeks after removing hairs of experimental mouse (Balb/c). Fig. 10 is a state of mouse applied with the composition for 2 weeks after removing hairs of the same species with the same age. A definite growth of mouse hairs could be found by the naked eye.
   In Fig. 11, the lengths between the mouse applied with distilled water and the mouse applied with the composition were compared after removing hairs of the experimental mice (C3H). It was confirmed that the hairs (Light side) of mice applied with the composition were quickly grown. A distance of a graduated ruler means 1 mm.
Figs. 12 to 16 are photographs illustrating examples of applying the composition of the present invention to the human body.
   The compositions including the phospholipids with the concentration of 5 mg/ml were applied to volunteers showing hair loss, and then the results were observed. One drop (50 to 60 ml, respectively) of the composition was applied to 2 cmx2 cm of the hair loss region twice a day, and rubbed for about 20 seconds. Fig. 12 is an appearance of volunteer A before applying; Fig. 13 is the appearance at 15 weeks after applying; Fig. 14 is the appearance at 20 weeks after applying; Fig. 15 is an appearance of volunteer B before applying; and Fig. 16 is the appearance at 15 weeks after applying. An effect of the composition could be easily and clearly observed even with the naked eye.

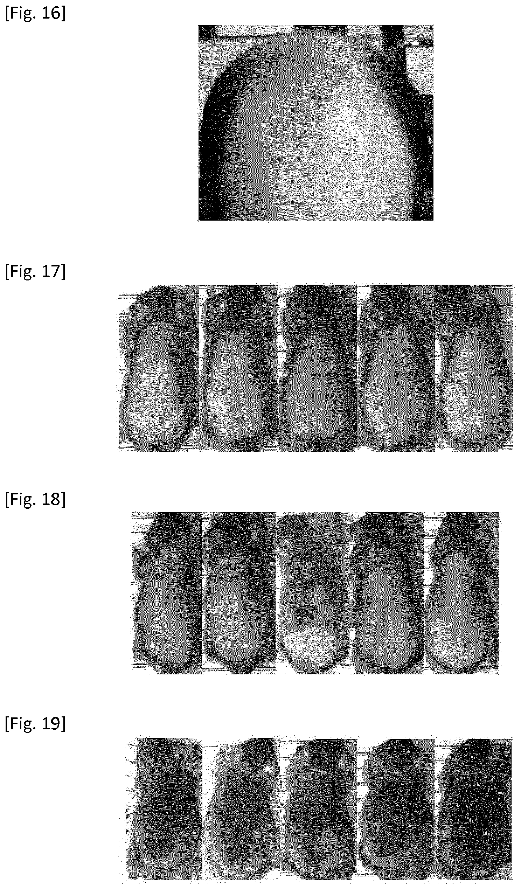

Figs. 17 to 24 are photographs illustrating an effect of the composition of the present invention including the combination of organic acids with various concentrations.
   An effect of hair growth was numerically converted by calculating the ratio of the hair growth area based on the hair removed area of the experimental mice (C3H) (Experimental Example 4: Hair growth effect experiment); see the content (organic acid effect and soybean lecithin effect) of Animal Experiment 3 and Table).

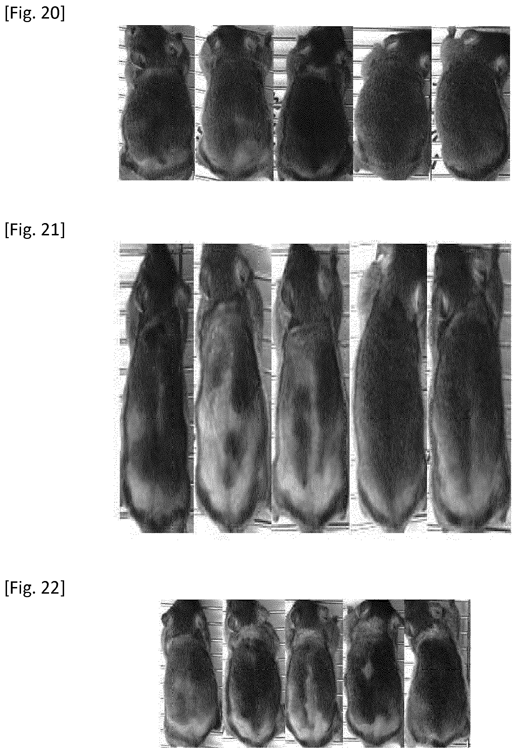

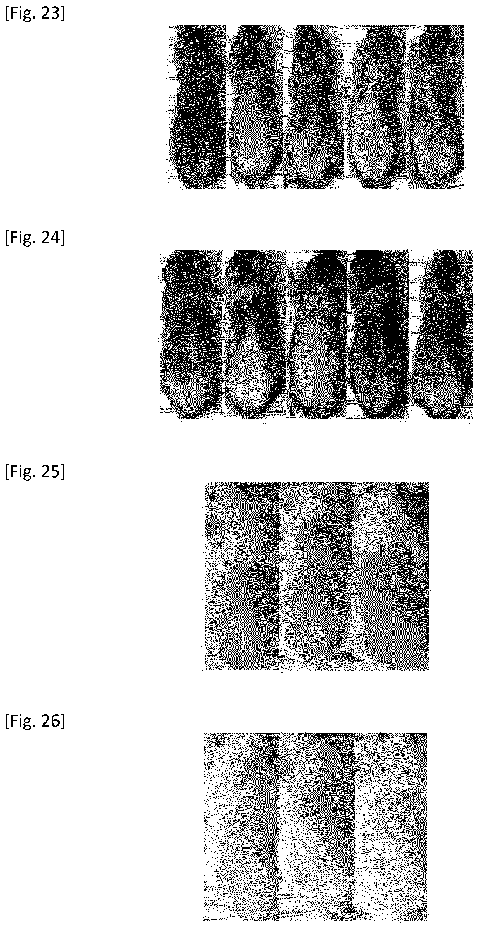

   Fig. 17 is an experimental group applied with only distilled water; Fig. 18 is an experimental group applied with only minoxidil (5%); Fig. 19 is an experimental group applied with the phospholipids extracted from pig lung + 1.5 mM CaCl₂ + 2.5 mM citrate/citric acid (pH 5.8); Fig. 20 is an experimental group applied with the phospholipids extracted from pig lung + 1.5 mM CaCl₂ + 5 mM citrate/citric acid (pH 5.8); Fig. 21 is an experimental group applied with the phospholipids extracted from pig lung + 1.5 mM CaCl₂ + 7 mM citrate/citric acid (pH 5.8); Fig. 22 is an experimental group applied with the phospholipids extracted from pig lung + 1.5 mM CaCl₂ + 5 mM citrate/citric acid + 5 mM of malate/malic acid (pH 5.8); Fig. 23 is an experimental group applied with the phospholipids extracted from pig lung + 1.5 mM CaCl₂ + 2.5 mM citrate/citric acid + 2.5 mM of malate/malic acid (pH 5.8); and Fig. 24 is an experimental group applied with the phospholipids extracted from soy lecithin + 1.5 mM CaCl₂ + 5 mM citrate/citric acid (pH 5.8). It was confirmed that when the concentration of citric acid was 5 mM as the organic acid including the composition, the most effective result was observed, and in the case of the combination with organic acid (for example, malic acid) relating to other citric acid cycle exhibited a relatively excellent hair growth effect.
Figs. 25 to 27 are photographs illustrating an effect of divalent cation included in the composition of the present invention.
   Instead of citrate/citric acid (pH 5.8) with 5 mM concentration included in the composition, 5 mM succinate/succinic acid (pH 5.8) was used. After applying with such the composition, an effect of the changed composition on hair growth was observed. Fig. 25 is an experimental group applied with only distilled water; Fig. 26 is an experimental group applied with the composition including 1.5 mM CaCl₂ + 5 mM citrate/citric acid (pH 5.8) (application for 2.5 weeks); and Fig. 27 is an experimental group applied with the composition including 1.5 mM CaCl₂ + 5 mM succinate/succinic acid (pH 5.8). It was found that hair growth of mice applied with the composition including succinate was not regular and also has coarse texture as compared with the state of hair growth of mice applied with the composition including citrate. It could be confirmed that citrate was most preferable as organic acid included in the composition, and also succinate had an effect on stimulating hair growth.
Figs. 28 to 29 are photographs illustrating an effect of magnesium ion as divalent cation included in the composition of the present invention.
   The composition including 1.5 mM MaCl₂ instead of 1.5 mM CaCl₂ was applied to the experimental mice (Balb/c) for 2 weeks. Distilled water was applied to the control experimental animal group (Fig. 28), and 20 mg/ml of phospholipids extracted from pig lung, 1.5 mM MgCl₂, and 5 mM citrate/ were applied to the effect group (Fig. 29). The composition including magnesium ion exhibited different type of hair growth individually. It was found that calcium ion among divalent cation included in the composition stimulated most effectively hair growth, and magnesium ion also effected on the hair growth.

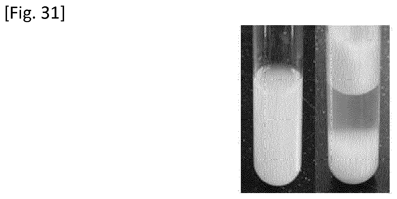

Figs. 30 and 31 are experiments relating to a dosage form of the composition of the present invention.
   A middle test tube in Fig. 30 exhibits phenomenon of aggregating purified phospholipids due to 1.5 mM calcium chloride. A right test tube in Fig. 30 exhibits a state of not aggregating the phospholipids due to calcium because of adding 5 mM citric acid/citrate (pH 5.8). A left test tube in Fig. 30 is a turbid result of distributing the composition in distilled water. From the above result, it could be confirmed that the organic acid included in the composition plays an important role in stimulating hair growth, but the organic acid is an essential factor for maintaining a state of turbid homogenized composition. A formation of liposome could be decided by confirming a formation of translucent milk color solution in the distilled water. However, the solution (right test tube) mixed with 5 mM citric acid/citrate + 1.5 mM calcium chloride has a homogenized turbid state, but there are opaque floccules-type particles so that it could be confirmed that the liposome was not formed (with referring to the photographs, it could be distinguished through an opaque right test tube).
   Fig. 31 is a result of trying liposome formation through vigorous vortex after forming lipid film on a circular flask in order to form liposome structure using the phospholipids extracted from lung. The soy lecithin passed through the same process maintained a translucent liposome solution state (Left side in Fig. 31), but when the phospholipids extracted from animal lung was left for 1hour, there were much bubble and many sedimentations (Right side in Fig. 31). It can be decided that the conditions of phospholipids and solution used for the present invention are not good for forming liposome.

Hereinafter, the present invention will be described in more detail with referring to a non-limitation Examples.

However, the following Examples are only for illustrating the present invention, but the range of the present invention is not limited to the following Examples.

### Example 1: Purification of phospholipids from pig lung

A pig was killed in a slaughter house that is certified by hazard analysis critical control point (HACCP), and immediately, lung tissue was obtained and then stored in an ice-water bath. After the slaughter, the tissue of pig lung was used within 5 hours, or in the case of not using immediately, it was stored at - 20 degrees, and then thawed it and then used, if necessary.

An extraction process of phospholipids is as follows:
1). Grinding a pig lung tissue; 2). Removing blood from the lung tissue; 3). Precipitating the total lipid; 4). Extracting the total lipid with organic solvent; 5). Removing neutral fats using neutral solvent; 6). Purifying using silica column in order to remove the remained neutral fats, and peptides and proteins; and 7). Removing the organic solvent and drying.

### 1). Grinding a pig lung tissue

Hard tissues (for example, trachea) or thick blood vessels (for example, aorta and pulmonary artery) were removed from the pig lung tissue, cut in a size of about 4x4x4 cm, and then ground the pig lung tissue using a grind mill. A cold saline solution (700 ml) was added to the ground tissue (250 g), and then ground further for 1 minute using a metal-blade blender (HMF-390, Hanil Electric, Korea).

### 2). Removing blood from the lung tissue

The lung tissues ground with a mixer was placed in an ice-water bath for 1 hour to cool down. The cold lung tissues were centrifuged at 4 degrees and 4,500 g for 40 minutes (Component-R, Hanil Electric, Korea) to collect precipitate and then remove the diluted blood supernatant.

### 3). Precipitating the total lipid

A storage solution (70 mM NaCl) including 5 mM CaCl₂ was added to the obtained precipitate, and then placed to an ice-water bath for 1 hour to cool down. The cold sample was centrifuged at 4 degrees and 4,500 g for 45 minutes to obtain a precipitate. Calcium ion was used to induce an aggregation of the unique proteins of lung tissue and phospholipids and then easily precipitate even at low centrifugation weight values (g).

### 4). Extracting the total lipid with organic solvent

The mixed solution of dichloromethane:ethanol (ethyl alcohol) (2:1 volume ratio) that was four-time volume was poured to the obtained precipitate to remove an aqueous materials solution and then obtain an organic acid-dissolvable material (the total lipid including phospholipids). The solvent was removed by using a vacuum rotary evaporator and then the lipid materials included were concentrated.

### 5). Removing neutral fats using neutral solvent

The concentrated phospholipids-contained fraction was again dissolved in chloride methylene, and then applied to a silica column to remove cholesterol, triacylglycerol, fatty acids, and the like included in the sample, and then obtain pure phospholipids. A glass column with the ratio of diameter and height of 1:4 was used, and Merck 230-400 mesh was used as a silica gel. A non-polar or neutral solvent was initially used, and a developing solvent having high degree of polar was gradually used to elute first neutral fats and then finally elute polar fats, thereby easily purifying. Acetone that was 1 volume of silica gel, and chloride methylene that was 1 volume of silica gel were sequentially used, and in order to obtain phospholipids, chloride methylene:ethanol:water (6:8:1 volume ratio) that was 7 volumes of silica gel was used to run the column. The eluted fractions were applied to a thin layer chromatograph (TLC) to confirm the elution of phospholipids. The lipid fractions with Rf values of 0.3 to 0.7 (phospholipid Rf value) were selected and then obtained from the thin layer chromatograph. Chloroform: methanol:water (65:25:4 volume ratio) were used as the thin layer chromatography developing solvent.

### 6). Purifying using silica column in order to remove the remained neutral fats, and peptides and proteins

The organic solvent was removed from the finally obtained phospholipid fractions using a vacuum rotary evaporator. Again, pure ethanol was added to the obtained phospholipid solids, and then subjected to the re-evaporation process to remove the remained chloride methylene. Finally, the remained water was removed by using a freeze dryer at -85 degrees to obtain phospholipids in a type of powder.

### 7). Removing the organic solvent and drying.

A purity of purified phospholipids was confirmed by using a thin layer chromatography (TLC). 5% sulfuric acid dissolved in ethanol was sprayed on the lipids presented on the thin layer chromatography, and then subjected to charring at 190 degrees to develop colors. Chloroform:methanol:water (65:25:4 volume ratio) were used as a thin layer chromatography developing solvent. The developing solution had a strong polar, and neutral fats such as fatty acids and cholesterol had high running rate (0.8 or more of Rf value), but proteins or peptides were not moved from the dotting points (0.1 or less of Rf value). The phospholipids had Rf values of 0.3 to 0.76. It was confirmed that DPPC (dipalmitoyl phosphatidyl choline), PC (phosphatidyl choline), PS (phosphatidyl serine), PE (phosphatidyl ethanolamine), PI (phosphatidyl inositol), and PA (phosphatidic acid) were included in the phospholipids purified by using the thin layer chromatography (Fig. 1).

It was confirmed from the analysis of thin layer chromatography that the phospholipids (lecithin) extracted from lung had completely different components from the components included in soy lecithin or egg lecithin (Fig. 2).

It was confirmed whether or not proteins or peptides included in the sample were present by spraying 0.2% ninhydrine dissolved in ethanol on the thin layer chromatography. There were no proteins or peptides detected on the thin layer chromatography stained with ninhydrine (the results were not provided).

The concentration of total phospholipids included in the sample was quantitative-analyzed by using a modified Bartlett assay (Miller KJ. 1985. J. Bacteriol. 162: 263-270) with Fiske and Subbarow reagent. The content of DPPC, unique phospholipids included in lung included in the sample was quantitative-analyzed by using HPLC-ELS (Evaporative light Scattering) Detector system (Waters e2695 module system, USA). L-a-dipalmitoyl phosphatidyl choline (Sigma Chem., Co. USA) was purchaued to use for a concentration analysis. The content of DPPC included in the total sample was confirmed by about 32% (the results were not provided).

### Example 2: Preparation of composition

Since the purified phospholipids were presented in a type of powder, in order to apply to epidermis, the phospholipids were dispersed in water. After distributing in water, the phospholipids were autoclaved at 121 degrees and a pressure of 15 lb/cm² for 15 minutes. Divalent cation (calcium chloride or magnesium chloride) and organic acids (citric acid, maleic acid, and the like), that were used as a co-factor, were made in a concentration solution, autoclaved respectively, added to the distributed phospholipids solution, and mixed to be required concentration. A preparation of organic acid was to be a buffer solution having pH 5.8 through mixing acid form (for example, citric acid) and sodium salt form (for example, citrate tri-sodium salt). In order to verify effectiveness, the used concentration of phospholipids was 5 mg/ml, 10 mg/ml, and 20 mg/ml, respectively, the concentration of divalent cation was 1.5 mM, and the concentration of organic acid (the sum of concentrations of organic acid and organic acid salt) was to be finally 5 mM.

### Experimental Example 1: Test for hair growth effect: Animal Test 1 (Observation of hair growth effect)

In order to prepare animal models with an atopic dermatitis, or a contact dermatitis and allergic dermatitis, After 1 week of sensitizing with DNFB, DNFB was applied to the backs of mice total 10 times per 2 weeks to induce an artificial allergic dermatitis. Methods of administrating the materials showing effectiveness, and a treating agent, and preparing the expressions of animal models were performed according to the previous published document (Inagaki et al. 2006, Eur J Pharmacol 546:189-196). Since applying DNFB five times, the composition of the present invention, Tacrolimus ointment (trande name, Protopic, Astella Pharma Korea, Inc.), and dexamethasone (Sigma Chem. Co. USA, 0.1% solution dissolved in ethanol) were applied to the backs of mice occurred with inflammation twice a day in the amount of 60 ml, respectively. At 10 days after applying, the mice tissues were collected to test experiments.

An active hair follicle growth was observed in the mice tissues applied with the composition of the present invention (the concentrations of phospholipids of 10 mg/ml (Fig. 7) and 20 mg/ml (Fig. 8)), and was no observed in the mice tissues applied with dexamethansone (Fig. 6). During the process of wounding the backs of mice, the phenomenon that new hair follicles were formed by differentiating the stem cell of basal cell membrane into hair follicle cells, and denting was observed in the mice tissues applied with Tacrolimus (Fig. 5), but there were growth of hair follicle cells under the basal cell membrane of the mice tissues applied with the present composition. The results are at the base that is capable of deducing that the composition of the present invention has a function of hair growth, rather than a function of prevention of hair loss.

### Experimental Example 2: Test for hair growth effect: Animal Test 2 (Test of growth rate of hair growth)

Male experimental mice (5-week age, balb/c) of the same age were purchased (Oriental Bio, SungNam-si, Kyunggi-do, Korea), and accommodated under the new circumstance for 1 week. Food and water were freely supplied and circadian rhythm was adjusted by adjusting a lights out of a.m. 8 and lights on of p.m. 8. Humidity, temperature, and air cleaning and circulation were adjusted at 25 degrees and 50% humidity using an animal breeding machine (DJ-201, Daejong Lab, Seoul, Korea) in order for breeding. Five mice per a group were tested.

After removing hairs of male mice having the same age (6 age of weeks), the composition of the present invention was applied and then hair growth was observed. As a control group, distilled water of the same amount instead of the composition was applied and then rubbed in the same times (Fig. 9). The composition was simply prepared by mixing 20 mg/ml of the phospholipids with the solution including 1.5 mM CaCI2 and 5 mM of citric acid/citric acid salt (citric acid, free-salt and citrate tri-sodium salt, pH 5.8). 60 ml of the composition was applied twice a day for 2 weeks (total 10 days except Saturday and Sunday) (Fig. 10). After 2 weeks, there was significantly a difference between two groups (Figs. 9 and 10).

In addition to Balb/c experimental mice, a growth rate of hair growth was compared by using C3H experimental mice. The lengths were compared by pulling mice hairs applied with only distilled water and the composition. After applying for 2.5 weeks, there was significantly difference of the hair growth length for two experimental groups. Fig. 11 is hair lengths pulled from two experimental groups, and the calibration indicates a distance of 1 mm.

### Experimental Example 3: Test for hair growth effect: Volunteer test

Volunteers whose hair loss are in progress or are greatly progressed were selected and then the composition of the present invention including the concentration of phospholipids of 5 mg/ml was applied. The volunteers whose a drug that can affect hair loss are administrated or applied on scalp were excluded. 13 volunteers were selected and the test for the volunteer was performed. 7 volunteers were participated in the test until completing for 15 to 20 weeks. An average age was 37. One drop (about 50 to 70 ml) of the composition of the present invention was dropped on the area of hair loss (2cmx2cm) twice a day, and then rubbed for 20 seconds. There were no abstinences or recommends relating to use of shampoo, use of detergent, food, alcohols, or smoking. The composition was applied during daily life. There were four questions the volunteers after applying the composition, in which the questions are related to the change of a decrease of hair loss, an increase of hairs, a growth rate of hairs, and a thickness of hairs. The volunteers selected themselves their satisfaction levels, the biggest 0 to 5 points relating to each of the questions.

**[Table 1]**

| | Decrease of hair loss | Change of hair thickness | Increase of hairs | Growth rate of hairs |
|---|---|---|---|---|
| Volunteer self evaluation score (0 to 5 points, n=7) average | 3.5 | 2.9 | 3.0 | 3.7 |

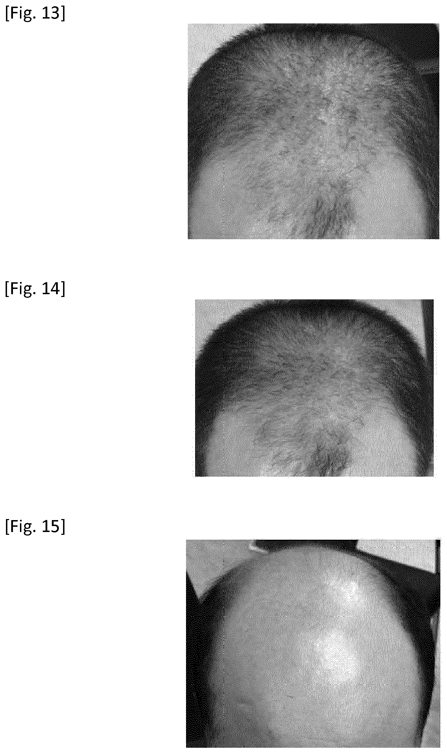

After applying the composition, the results obtained through the volunteers evaluated themselves were subjective opinions thereby not assuring universal accuracy. However, it could be found that the satisfaction degree of the volunteers about the effect of the composition were high. From the evaluation results of the volunteers, it could be confirmed that the present inventors deduced the result that the composition was effective for stimulating hair growth, not preventing hair loss. The result applied to the volunteer could be easily confirmed by the naked eye through the photographs (Figs. 12 to 16).

### Experimental Example 4: Test for hair growth effect: Animal Test 3 (Organic acid effect and soy extracted phospholipid effect)

The experiment was performed in order to confirm the effect of organic acid (for example, citric acid free-salt and citrate tri-sodium salt) included in the composition of the present invention on hair growth. In addition, the composition was prepared by using soy lecithin (phospholipid extracted from soy, #86015-1201, Junsei Chemical, Japan), and then effect of the composition on hair growth was compared. C3H/HeNCrljBgi species of 7 ages of weeks (Orient Bio, Sungnam, Korea) were purchased as the experimental mice, and then used for the test. In order to exhibit a significant difference of the experimental result, 60 ml of minoxidil (5% solution, Minoxyl, Hyndaipharm. Korea) was applied to all the mice (except Group 1), and then after 30 minutes, the composition of the present invention was applied. The composition of the present invention was applied for 2.5 weeks, and 60 ml of the composition was applied on the backs of the hairs-removed mice twice a day. The concentration of the applied phospholipids was 20 mg/ml. As a measurement of hair growth in the mice, the pictures of the mice of each experimental group were taken, respectively, and then the ratio of the hair growth area in the hairs-removed area was calculated. The results are shown in the following Table.

**[Table 2]**

| Animal Group | Applied component | Hair growth area |
|---|---|---|
| Group 1 | Distilled water application (Fig. 5a) | 0 |
| Group 2 | Minoxidil (5%) application (Fig. 5b) | 10.2 |
| Group 3 | Phospholipids extracted from pig lung+1.5mM CaCl₂+2.5mM citrate (Fig. 5c) | 77.4 |
| Group 4 | Phospholipids extracted from pig lung+1.5mM CaCl₂+5mM citrate (Fig. 5d) | 83.4 |
| Group 5 | Phospholipids extracted from pig lung+1.5mM CaCl₂+7mM citrate (Fig. 5e) | 59.5 |
| Group 6 | Phospholipids extracted from pig lung+1.5mM CaCl₂+5mM citrate+5mM malate (Fig. 5f) | 73.9 |
| Group 7 | Phospholipids extracted from pig lung+1.5mM CaCl₂+2.5mM citrate+2.5mM malate (Fig. 5g) | 36.7 |
| Group 8 | Phospholipids extracted from soy (soy lecithin)+ 1.5mM CaCl₂+5mM citrate (Fig. 5h) | 27.9 |

In order to speed up hair growth rate of experimental mouse, it is preferable to use the composition extracted from pig lung, and it was found that use of 5 mM of the concentration of citrate in the composition given the most effective results (Fig. 20). It was confirmed that the phospholipids extracted from soy (soy lecithin) helped to stimulate mouse hair growth, but it was considered that such an effect of the soy lecithin did not have the great meanings as compared with an effect of the phospholipids extracted from pig lung on stimulating hair growth.

Furthermore, an effect of the composition by further adding malate/malic acid was investigated. It could be confirmed that organic acid relating to TCA cycle of mitochondria helped hair growth. In addition, the result from which the hypothesis that the physiological activity deeply relating to the lipid metabolism of mitochondria may involve in hair growth could be deduced (Figs. 17 to 24).

### Experimental Example 5: Test for hair growth effect: Animal Test 4 (Comparison between citrate and succinate)

Instead of citrate included in the composition of the present invention, succinate di-sodium salt/succinic acid (pH 5.8), other representative organic acid of citric acid cycle of mitochondria in the same concentration (5 mM) was added to investigate an effect on hair growth. Balb/c experimental mice with 9 age of weeks were used by using the same method as described above for 2.5 weeks as an application period. Succinate exhibited a stimulation of mice hair growths like citrate (Fig. 27). However, the composition including succinate exhibited irregular hair growth and also hairs with coarse texture as compared with the composition including citrate (Fig. 26). However, the mice applied with the composition including succinate sometimes partially exhibited more rapid hair growth as compared with that of the composition including citrate. It was confirmed that the organic acid included in the composition was essential for preventing an aggregation phenomenon of the phospholipids due to calcium and at the same time was an important factor for controlling hair growth.

### Experimental Example 6: Test for hair growth effect: Animal Test 5 (Effects of divalent cation, calcium ion, and magnesium ion)

Instead of calcium ion, divalent cation, included in the composition of the present invention, an effect of magnesium ion was investigated on mouse hair growth rate. Balb/c experimental mice with 6 ages of weeks were used, and 50 ml of the composition was applied on the backs of mice twice a day for 3 weeks (15 days). The composition was prepared to include 20 mg/ml of phospholipids, 1.5 mM MaCl₂, and 5 mM of citric acid/citrate sodium salt (pH 5.8).

From the results of observing after applying the composition for 3 weeks, it was confirmed that the magnesium ion also stimulated hair growth rates of mice. However, it did not show the satisfactory effect as compared with the effect of the composition including calcium ion. The composition including calcium ion exhibited regular hair growth in all the mice of the experimental groups, while the composition including magnesium ion exhibited irregular hair growth depending on the individuals. From the experiments, it could be concluded that calcium ion was most suitable for stimulating mouse hair growth as divalent cation included in the composition, and magnesium ion had potential to play a role in stimulating mouse hair growth (Figs. 28 and 29).

### Experimental Example 7: Test for observing dosage form of composition (combination of calcium ion and organic acid)

The composition of the present invention includes phospholipids extracted from lung, calcium chloride, and organic acid (citrate + citric acid salt). Especially, the organic acid is an important factor for speeding up hair growth, and at the same time, plays a role in preventing an aggregation of phospholipids due to calcium ion. This experiment was performed by verifying that the organic acid included in the composition inhibits an aggregation phenomenon of phospholipids due to calcium ion. It could be clearly determined that the organic acid is essentially included in the composition, so that the skin application can be easily performed.

A state of distributing 10 mg/ml of phospholipids in distilled water (Left side in Fig. 30), a state of only adding calcium chloride to the phospholipid-distributed solution to be a concentration of 1.5 mM (Middle in Fig. 30), and a state of adding 1.5 mM of calcium chloride and 5 mM of citrate/citric acid to the phospholipid-distributed solution (Right side in Fig. 30) were prepared. There were an aggregation phenomenon of phospholipids in the test tube including only calcium chloride, and a state of uniformly distributing the phospholipids in the test tube including organic acid and calcium together.

A condition for forming liposome of phospholipids extracted from pig lung was investigated. Chloroform:methanol (1:2 volume ratio) were added to the phospholipids extracted from lung, and then a thin layer lipid film was formed on the wall of flask using a vacuum rotary evaporator (Eyela N-1000, rotary vacuum evaporator, Japan). 1.5 mM of calcium chloride and 5 mM of citric acid/citrate tri-sodium salt (pH 5.8) that are a composite of the present patent were added to the round bottom flask formed with the phospholipid film, and then vigorously shaken for 30 minutes to form liposome. At the time of forming liposome, the flask was maintained to about 60 degrees by heating at regular intervals. The phospholipids extracted from lung exhibited a bubble formation different from the soy-extracted phospholipids (soy lecithin), and when maintaining the phospholipids extracted from lung for 1 hour at a room temperature, the phospholipids extracted from lung was easily precipitated. It was decided that the condition applied for the composition of the present invention was not advantageous. This is because the phospholipids extracted from pig lung has high saturation degree, so that the fluidity of hydrocarbon tails was low, and the head part of phospholipids had high polarity, so that they exhibited strong electrostatic repulsion to each other. It can be expected that the composition of the present invention recommends that the phospholipid extracted from health animal lung was used in a simply turbid state, and the composition including phospholipids having such a non-liposomal structure can be easily mixed with sebum with high viscosity, so that the sebum viscosity can be quickly decreased, and easily removed.

## Claims

1. A composition comprising phospholipids as an effective component, wherein the phospholipids are extracted from an animal lung, for use in the therapy of hair loss by stimulating hair growth.

2. The composition according to claim 1, wherein the phospholipids are extracted from a cow or pig.

3. The composition according to any one of claims 1 to 2, wherein the phospholipids are selected from the group consisting of dipalmitoylphosphatidylcholine, phosphatidylcholine, phosphatidylserine, phosphatidic acid, and phosphatidylinositol.

4. The composition according to claim 1, further comprising one or more component selected from the group consisting of divalent cations, and carboxyl group-containing organic acid or salt thereof.

5. The composition according to claim 1, wherein the composition is a pharmaceutical composition.

6. The composition according to claim 1, wherein the composition is a cosmetic composition.

7. The composition according to claim 6, wherein the cosmetic composition is a hair tonic, a hair cream, a hair lotion, a hair shampoo, a hair rinse, a hair conditioner, a hair sprayer, a aerosol, pomade, powder, or gel.

8. Non-therapeutic use of the composition according to claim 1 for preventing hair loss or stimulating hair growth.

## Patentansprüche

1. Zusammensetzung umfassend Phospholipide als wirksame Komponente, wobei die Phospholipide aus einer Tierlunge extrahiert werden, zur Verwendung in der Behandlung von Haarausfall durch Stimulierung des Haarwachstums.

2. Zusammensetzung gemäß Anspruch 1, wobei die Phospholipide aus einer Kuh oder einem Schwein extrahiert werden.

3. Zusammensetzung gemäß einem der Ansprüche 1 oder 2, wobei die Phospholipide ausgewählt sind aus der Gruppe bestehend aus Dipalmitoylphosphatidylcholin, Phosphatidylcholin, Phosphatidylserin, Phosphatidsäure und Phosphatidylinositol.

4. Zusammensetzung gemäß Anspruch 1, weiterhin umfassend eine oder mehrere Komponenten, ausgewählt aus der Gruppe bestehend aus zweiwertigen Kationen und einer Carboxylgruppen enthaltenden organischen Säure oder einem Salz davon.

5. Zusammensetzung gemäß Anspruch 1, wobei die Zusammensetzung eine pharmazeutische Zusammensetzung ist.

6. Zusammensetzung nach Anspruch 1, wobei die Zusammensetzung eine kosmetische Zusammensetzung ist.

7. Zusammensetzung nach Anspruch 6, wobei die kosmetische Zusammensetzung ein Haartonikum, eine Haarcreme, eine Haarlotion, ein Haarshampoo, eine Haarspülung, ein Haar-Conditioner, ein Haarspray, ein Aerosol, eine Pomade, ein Pulver oder ein Gel ist.

8. Nicht-therapeutische Verwendung
der Zusammensetzung gemäß Anspruch 1 zur Verhinderung von Haarausfall oder zur Stimulierung des Haarwachstums.

## Revendications

1. Composition comprenant des phospholipides en tant que composant efficace, dans laquelle les phospholipides sont extraits d'un poumon d'animal, pour une utilisation dans le traitement de la perte de cheveux par stimulation de la croissance des cheveux.

2. Composition selon la revendication 1, dans laquelle les phospholipides sont extraits d'une vache ou d'un cochon.

3. Composition selon l'une quelconque des revendications 1 à 2, dans laquelle les phospholipides sont sélectionnés dans le groupe constitué par la dipalmitoylphosphatidylcholine, la phosphatidylcholine, la phosphatidylsérine, l'acide phosphatidique et le phosphatidylinositol.

4. Composition selon la revendication 1, comprenant en outre un ou plusieurs composants sélectionnés dans le groupe constitué par les cations divalents, et un acide organique contenant un groupe carboxyle ou un sel de celui-ci.

5. Composition selon la revendication 1, dans laquelle la composition est une composition pharmaceutique.

6. Composition selon la revendication 1, dans laquelle la composition est une composition cosmétique.

7. Composition selon la revendication 6, dans laquelle la composition cosmétique est un stimulant capillaire, une crème capillaire, une lotion capillaire, un shampooing pour les cheveux, un rinçage capillaire, un baume démêlant pour les cheveux, une laque pour les cheveux, un aérosol, une pommade, une poudre ou un gel.

8. Utilisation non thérapeutique de la composition selon la revendication 1 pour la prévention de la perte de cheveux ou la stimulation de la croissance des cheveux.
